# EUROPEAN PATENT APPLICATION

(11) **EP 4 512 339 A1**
(43) Date of publication of application: **26.02.2025**
(21) Application number: 23192332.7
(22) Date of filing: 21.08.2023
(51) Int. Cl.: A61B 6/03, A61B 6/00, G06T 7/246

(54) **SYSTEM AND METHOD FOR MONITORING A SUBJECT**

(71) Applicant: Koninklijke Philips N.V., 5656 AG Eindhoven (NL)
(72) Inventor: FRERKING, Lena Christina, Eindhoven (NL); SCHMITT, Holger, Eindhoven (NL); SENEGAS, Julien Thomas, Eindhoven (NL); NETSCH, Thomas, Eindhoven (NL); DESHPANDE, Hrishikesh Narayanrao, Eindhoven (NL)
(74) Representative: Philips Intellectual Property & Standards

(57) **Abstract**

The invention provides concepts pertaining to monitoring a subject positioned on a subject couch during a medical scan. In particular, detecting movement of a subject during a scan is of high importance to ensure that the subject is not moving during the scan, in order to prevent imaging artifacts. Thus, some systems employ cameras to detect movement of the subject during the scan, and provide motion alerts when motion is detected. However, the detected motion corresponds to movement of the visible surface of the subject, rather than actual movement of the body. Cavities/gaps may exist between the body surface of the subject and visible surface (e.g., clothes, blankets), so visible movement may relate to movement of only the visible surface (e.g., due to airflow). Therefore, a camera image of the visible surface and a scanner image of a body surface of the subject are leveraged in order to generate a cavity map describing gaps between the body surface and visible surface. Then, when motion is detected, generation of the motion alert is also based on the cavity map to reduce the occurrence of false alerts.

## Description

### FIELD OF THE INVENTION

This invention relates to the monitoring of a subject during a medical scan, such as a CT scan.

### BACKGROUND OF THE INVENTION

During a CT examination, the subject is typically left alone in the CT room until the completion of the exam. Even though CT scans are rather short in time, multiple scans can be done in sequence, with or without contrast agent administration, so that a typical examination can take up to 15 minutes. During this time, it is highly desirable to continuously monitor the subject and check that they feel safe and comfortable and can follow the instructions. It is especially of high importance to ensure that the subject is not moving during the scan, in order to prevent imaging artifacts.

To monitor the subject, cameras may be provided that can be used to detect potential motion events during an acquisition and alert the operator that the scan might be affected and contains motion artifacts. Such cameras, which may be attached to the CT gantry, track the deformations of the visible surface of the scene, located on the subject couch. However, a deformation of the visible/outer surface may not necessarily conform with actual motion of the subject body.

In particular, it may be that a thin fabric (e.g., clothes or a blanket) which covers the subject, moves due to ventilation while the subject remains still. In this case, the camera may detect a motion event, which leads to a false alert to the operator. Conversely, it is possible that the subject moves underneath a layer of clothes or blankets, but the movement does not give rise to any motion of the visible surface. This may occur if there is a cavity underneath the visible surface that is not visible for the camera. In such cases, a significant motion event may not be recognized by the camera even though there may appear artifacts in the reconstructed image.

### SUMMARY OF THE INVENTION

The invention is defined by the claims.

According to examples in accordance with an aspect of the invention, there is provided a system for monitoring a subject positioned on a subject couch during a medical scan.

The system comprises:
a camera for generating a camera image of a visible surface corresponding to the subject on the subject couch at a point in time;
a scanner for generating a scanner image of a body surface of the subject on the subject couch at the point in time; and
a processor for processing the camera image and the scanner image, and configured to:
   generate a cavity map describing distances between the visible surface and the body surface in a plurality of locations based on a comparison of the camera image and the scanned image;
   detect motion of the visible surface of the subject in at least one of the plurality of locations during the scanning session; and
   generate a motion alert signal indicating movement of the subject based on the detected motion and the cavity map.

This system alerts a scan operator to any movement of the visible surface of the subject (e.g. exposed surface of the subject or clothes/a blanket covering the subject) that corresponds to actual movement of the body of the subject. Movement of the body of the subject may give rise to artifacts in the image produced during the medical scan, whereas movement of, for example, clothes of the subject will not have any impact. Accordingly, the occurance of false alarms may be avoided, saving time and cognitive effort of medical professionals and improving comfort of the subject.

That is, a cavity map is generated describing gaps/distances between the actual body/tissue surface of the subject and the visible surface. Then, when movement of the visible surface is detected, a motion alert signal is generated based on both the detected movement and the cavity map. So, if the movement of the visible surface occurs in the vicinity of a large cavity, then a motion alert signal may not be generated, as this may simply correspond to movement of the visible surface (e.g. due to airflow). Whereas, if no such cavity exists (e.g., the clothes hugging the subject tightly, or movement of exposed tissue surface), then a motion alert signal will be generated as a confidence of movement is high.

Furthermore, the detection of cavities enables a calculation of how reliable motion detection is in a specific region. Indeed, if there is motion inside a cavity, which can not be detected by the camera, then the knowledge about the cavity does not help to actually identify motion there. However, a user may be informed that there is a large cavity in a specific area, and therefore may be able to prioritize assessment of images captured by the scanner to retrospectively identify image artifacts as a result of movement. That is, an area may be marked as having uncertain motion detection, and an operator may immediately have a closer look at the highlighted area.

In some embodiments, the camera may be further configured for generating camera images of the visible surface of the subject during the medical scan. In this case, the processor may be further configured to detect motion of the visible surface of the subject based on the camera images of the visible surface of the subject during the scanning session.

Put another way, the camera used to generate the cavity map may be the same camera/camera system as the means for detecting motion of the visible surface during the medical scan. Alternatively, a separate camera system may be utilised, or perhaps a motion sensor embedded in the visible surface (e.g. a motion sensor integrated in clothes/a blanket of the subject).

In some embodiments, the scanner image may further include a covering surface corresponding to the subject on the subject couch.

Some types of scanner images (e.g., CT images) may also include an image of a covering (e.g., clothing, blankets, protection devices) on the subject. Thus, both the covering surface (consisting of clothes and blankets, etc.) corresponding to the visible surface, and the body surface can be detected (segmented) on the scanner image. As a result, a comparison between the scanner image and the camera image may be more accurate, as a mapping between the images may be more accurate.

The processor is for example further configured to determine a motion confidence value indicative of a likelihood of movement of the body surface of the subject based on the detected motion and the cavity map. Generating the motion alert signal may be based on the motion confidence value.

Of course, it may not be completely clear whether the movement of the visible surface does or does not correspond to actual movement of the subject body. Therefore, a confidence value may be generated describing a degree of confidence in a judgement to whether subject movement has occurred. This could be compared to a (potentially adjustable, according to operator preferences) requirement to determine whether to generate a motion alert signal. This may enable a tuning of the sensitivity of the system to movement of the visible surface, so that a more accurate determination of movement/appropriate alert signal may be generated.

The processor may also be configured to generate the motion alert signal responsive to the motion confidence value satisfying a predetermined requirement.

In some embodiments, the processor may be further configured to quantify an amount and/or direction of motion of the visible surface. The processor may then be configured to generate the motion alert signal further based on the amount and/or direction of motion.

The magnitude and direction of motion of the visible surface may also provide relevant information to determine whether actual body movement has occurred (in addition to location of motion and cavities in the vicinity of the motion). For example, motion of the visible surface in a direction unnatural for movement of clothes/a blanket may confidently indicate body movement. In addition, large sharp movements may indicate actual body movement. Therefore, including this information may lead to more accurate determinations of subject motion, and more appropriate generation of motion alert signals.

For example, the detected motion of the visible surface of the subject is located at a region of the subject corresponding to a scan region of the medical scan.

Subject motion in a location outside the scan region is less relevant to success of the scan than motion within the region. Therefore, motion alert signals may only be generated when the visible surface moves within the scan region. This may reduce effort and cognitive load needed by a medical professional, as well as reducing the computational power required for the processor to effectively detect motion.

In some embodiments, the processor is further configured to generate a reliability map describing reliability of movement detection by the camera at each of the plurality of locations based on the cavity map, and wherein the processor is configured to generate the motion alert based on the detected motion and the reliability map.

The reliability map provides a means for translating the cavity map into a meaningful indicator of the correlation between visible surface movement and body surface movement. That is, areas where the visible surface and the body surface are close may have a high reliability, whilst areas where they are distant will have low reliability. This may be a particularly useful tool for a medical professional/operator to quickly and easily determine/verify the presence or absense of actual subject movement.

Put another way, the reliability map provides a kind of heat map, highlighting areas/regions of the visible surface where motion may not be detected even for significant bodily movements beneath the surface. If there is motion in areas where there exist large cavities, the motion may not be detected by the camera. Thus, by detecting an area with a large cavity, a low level of reliability may be associated. Accordingly, an operator or user may be prompted to focus on the scan produced for these areas, as artifacts may be present in the canner image even without detected movement by the camera.

Furthermore, the processor may be further configured to identify regions of the subject corresponding to values of the reliability map failing to meet a predetermined condition, and generate an alert describing the identified regions.

Accordingly, an alert and/or pointer may be generated that highlights regions where motion may not be detected. Thus, the invention leverages the cavity map, not only to generate an alert when motion is detected with high confidence, but also to aid in detecting artifacts in scanner images where a camera is not able to detect movement.

Indeed, the detection of cavities enables the generation of a reliability map having a confidence associated with motion detection in different areas. This is based on the fact that if there is motion inside a cavity (which cannot be detected by the camera) then the knowledge about the cavity does not help to actually identify motion there. However, knowledge of the cavity helps to draw attention of the user or operator to this area. For example, if there is a huge cavity (indicated by a low confidence value) the area of the associated reconstructed slice of the scan, may be marked as unsure or unreliable by the alert highlighting regions. In this way the operator may immediately have a closer look at the region(s) while passing quickly by regions with a high confidence of motion detection.

The processor, for example, may be further configured to generate a visible surface map describing a shape of the visible surface of the subject based on the camera image, generate a body surface map describing a shape of the body surface of the subject based on the scanner image, and generate the cavity map based on comparing the visible surface map to the body surface map.

Thus, by segmenting and comparing images from the camera and the scanner (using known image analysis techniques), an accurate cavity map may be generated in a relatively simple, memory efficient, and computationally efficient manner.

Also, the processor may generate the visible surface map further based on the scanner image.

As above, the scanner image (e.g., CT image) may include the fabric covering the subject. Thus, the scanner image may also be segmented to provide information about the visible surface, and thus generate the visible surface map. This may improve an accuracy of mapping between the scanner image and cavity image, and thus improve an accuracy of the visible surface map.

In some embodiments, the processor may be further configured to register the camera image and the scanner image based on a camera calibration with respect to the scanner and/or a scanner calibration with respect to the camera.

The scanner image may be a 3D surview scanner image, and wherein the point in time precedes initiation of the medical scan.

A surview image is typically taken before conducting a scan in order to plan the final scan-box. Instead of conventional 2D surviews, which consist of either a coronal or a sagittal slice, it is also possible to acquire 3D surviews with minor modifications. This makes it possible to obtain more detailed information of the entire anatomy and can, therefore, be used to plan safety margins for the diagnostic scan with more accuracy.

It has been realized that these surviews can be leveraged in order to facilitate the invention without the need for additional scans. Indeed, surviews provide information important for generating a cavity map.

The invention also provides a method for monitoring a subject positioned on a subject couch during a medical scan. The method comprises:
generating a camera image of a visible surface corresponding to the subject on the subject couch at a point in time;
generating a scanner image of a body surface of the subject on the subject couch at the point in time;
generating a cavity map describing distances between the visible surface and the body surface in a plurality of locations based on a comparison of the camera image and the scanned image;
detecting motion of the visible surface of the subject in at least one of the plurality of locations during the scanning session; and
generating a motion alert signal indicating movement of the subject based on the detected motion and the cavity map.

The method may further comprise generating camera images of the visible surface of the subject during the medical scan. Detecting motion of the visible surface of the subject may be based on the camera images of the visible surface of the subject during the scanning session.

Further, the method may further comprise determining a motion confidence value indicative of a likelihood of movement of the body surface of the subject based on the detected motion and the cavity map. Generating the motion alert signal may be based on the motion confidence value.

Generating the motion alert signal may be performed responsive to the motion confidence value satisfying a predetermined requirement.

In some embodiments, the method may further comprise quantifying an amount and/or direction of motion of the visible surface. Generating the motion alert signal may be further based on the amount and/or direction of motion.

The invention also provides a computer program comprising computer program code which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a slice of a scan depicting a distance between visible surface and body surface of a subject;
Fig. 2 shows schematically an example of a medical scanner with a system for monitoring a subject positioned on a subject couch during a medical scan using the medical scanner; and
Fig. 3 shows a flow diagram of a method for monitoring a subject positioned on a subject couch during a medical scan.

### DETAILED DESCRIPTION OF THE EMBODIMENTS

The invention will be described with reference to the Figures.

It should be understood that the detailed description and specific examples, while indicating exemplary embodiments of the apparatus, systems, and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides concepts pertaining to monitoring a subject positioned on a subject couch during a medical scan. In particular, detecting movement of a subject during a scan is of high importance to ensure that the subject is not moving during the scan, in order to prevent imaging artifacts. Thus, some systems employ cameras to detect movement of the subject during the scan, and provide motion alerts when motion is detected. However, the detected motion corresponds to movement of the visible surface of the subject, rather than actual movement of the body. Cavities/gaps may exist between the body surface of the subject and visible surface (e.g., clothes, blankets), so visible movement may relate to movement of only the visible surface (e.g., due to airflow). Therefore, a camera image of the visible surface and a scanner image of a body surface of the subject are leveraged in order to generate a cavity map describing gaps between the body surface and visible surface. Then, when motion is detected, generation of the motion alert is also based on the cavity map to reduce the occurrence of false alerts.

By way of explanation, before acquiring a diagnostic scan (e.g., diagnostic CT scan), usually surview images are acquired. Surview images serve to plan the final scan-box. Instead of a conventional 2D surview image, which consist of either a coronal or a sagittal slice, it is possible to acquire 3D surviews. For CT scans, this requires a similar X-ray dose. 3D surview scans provide detailed information of the entire anatomy of the subject. Therefore, they can be used to plan safety margins for the diagnostic scan with more accuracy.

To monitor the subject/patient during a scan, it is possible to attach cameras to the scanner gantry and/or to a position in view of the subject during the scanning session. Said cameras can be used for surveillance of the subject, and may also be able to detect potential motion events during an acquisition/scanning session. Thus, cameras can be used to alert the operator that the scan might be affected, and may contain motion artifacts.

Typically, the cameras track deformations of the visible surface of the scene, located on the patient couch. That is, cameras record and track visible changes in the scene. However, a deformation of the visible, outer surface of the subject does not necessarily mean that the subject has moved. For example, it is possible that a thin fabric (e.g., clothes or a blanket) that covers the subject moves without requiring movement of the subject. The thin fabric may move, for example, due to ventilation while the patient itself keeps still. In this case the camera may detect a motion event, which leads to a false motion alert signal.

On the other hand, it is possible that the subject is, in fact, moving underneath a layer of clothes or blankets, but the clothes or blankets do not move and so the movement is not visible for the camera. This may occur if there is a cavity underneath the surface.

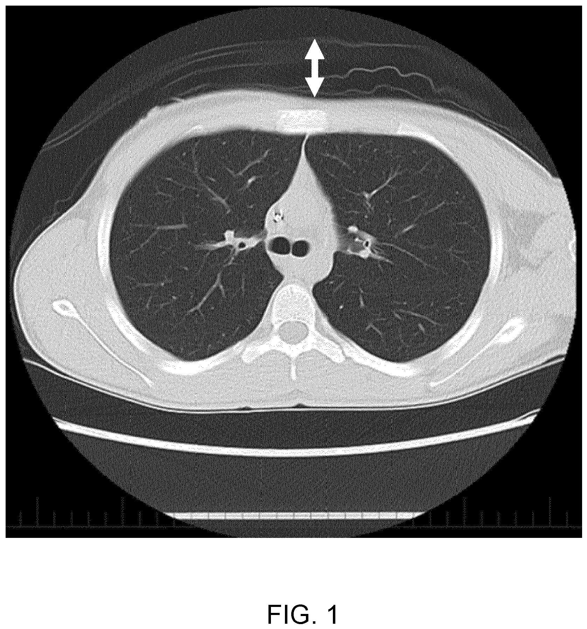

The presence of a gap beneath the visible surface is shown by the arrow in Fig. 1 which shows a scan of the chest. That is, the cavity between the visible surface and subject body surface is shown by the arrow. As a result, movement in this area may not be visible for a camera taking images of the surface. In such a case, a significant motion event may not be recognized by the camera even though the movement results in artifacts in the reconstructed image.

Embodiments of the invention aim to overcome this problem by combining information from a scanner image (such as a 3D surview image), and one or more cameras images to identify cavities between the visible surface corresponding to the subject and the actual body surface of the subject.

These detected cavities (in the form of a cavity map) may be considered for ranking the detected motion events. If the visible surface corresponding to the detected movement event is very close to the body surface according to the cavity map, detected motion events may be considered as very reliable. On the other hand, if motion occurs on at a visible surface that is not directly connected to the patient body (i.e., a large distance between the visible surface and the body surface), the motion may be caused by factors unrelated to movement of the subject (e.g., air flow). Such motion is highly unlikely to cause any image artifacts. In such cases, the detected motion is not reliable. Put simply, a map of cavities describing distances between visible surface and the body surface can be used as a base for weighted motion detection.

The invention may comprise the following features:
(i) At least one camera to monitor the subject and detect motion of the visible surface. The camera may preferably be gantry-mounted;
(ii) Use of the (gantry) camera and scanner image (e.g., a 3D surview) to project detected cavities between visible surface and body surface of the subject from 3D surview to 2D camera view representation;
(iii) The generation of a reliability map based on distances between the visible surface and body surface of the subject; and
(iv) Weighting of motion detections and adapting sensitivity for generating motion alert signals accordingly.

The impact of detected cavities on the statistical relation between true body surface movement and visible surface movement/deformation may be learned from corresponding data acquired in phantom and/or human experiments.

The camera image offers information, in particular a map, of the visible surface of the subject, including clothes, blankets that the patient is covered in and exposed tissue of the subject. In contrast a scanner image, such as a 3D surview, offers information of the structure behind the visible surface at the same time. The conformity of movement of the visible surface and the body surface of the subject dependents (at least in part) on the existence of cavities. In the scanner image, both the body surface of the subject and visible tissue layers may be visible, as seen in Fig. 1.

Hence, cavities can be identified by measuring the distance between the outermost part of the tissue (i.e., the visible surface), and the body surface of the subject. Since, human skin has characteristic Hounsfield Unit, HU, values, this distance may easily be found, e.g., by segmenting the entire body of the subject in the scanner image, segmenting the non-air layers around it, and then calculating the difference between the outermost parts of the segments. For the segmentation of the body surface outline of the subject using scanner images, and the visible surface contour from the camera image(s), convolutional neural networks may be employed.

To reiterate, the reliability of detected motion is strongly dependent on the existence of such cavities. Hence, the scanner image may be used to determine this reliability for individual regions.

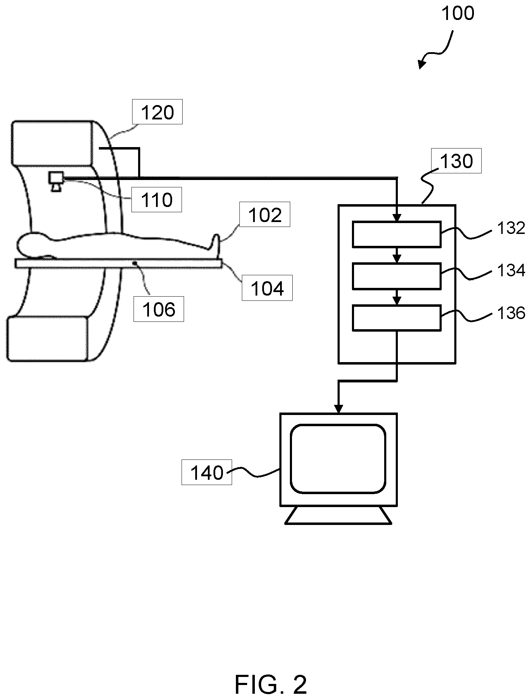

Fig. 2 shows schematically an example of a system 100 for monitoring a subject 102 positioned on a subject couch 104 during a medical scan. The system 100 includes a (medical) scanner 120 for acquiring a scanner image of a subject 102 at a point in time, and which additionally includes an imaging system (i.e. a camera 110) for capturing a camera image of the subject 102 at the point in time.

The scanner image is an image of the actual body of the subject 102, and which may or may not include fabric/cover over the subject 102. In any case, the shape of the body of the subject/actual position of the subject 102 may be readily observed from the scanner image. In contrast, the camera image is an image of the visible/outer surface of the subject 102. That is, the camera image shows exposed skin of the subject 102 and the clothes/blankets/outer coverings of the subject 102.

In preferred embodiments, the scanner image is an 3D surview scanner image acquired by the scanner 120. In this case, the point in time precedes initiation of the medical scan. 3D surview scanner images are images taken before the actual medical scan, and are generally used for calibration and determination of scan settings. Thus, no extra scanner images are required to implement the invention should the 3D surview scanner image be utilised.

Of course, the scanner 120 may also be adapted to generate scanner images throughout the medical scan for the purpose of performing the medical scan/examining the subject 102. Equally, the camera 110 may also be adapted to generate camera images throughout the medical scan. Said camera images may be generated for the purpose of monitoring the subject 102 during the medical scan.

However, separate systems (i.e., a different scanning system, and a different camera/motion detection system) may be used to image and monitor the subject 102 during the medical scan. For example, there may be different sensors that detect movement of the external surface of the subject 102, and the scanner 120 used to obtain the initial scanner image may be different to the scanner 120 used to obtain medical images of the subject 102. Nonetheless, in all embodiments of the invention the camera 110 generates a camera image of a visible surface corresponding to the subject 102 on the subject couch 104 at a point in time, and the scanner 120 generates a scanner image of a body surface of the subject 102 on the subject couch 104 at the point in time.

The operation of the system with a single camera 110 will be explained, but there may be multiple cameras positioned in a wide variety of locations suitable for viewing the subject. The camera 110 could be a color, monochrome, heat, or depth camera. It may use visible light or infrared light. The camera 110 may be calibrated with respect to the scanner 120 gantry.

The camera 110 may be mounted on the gantry as shown, but it may be mounted in another position relative to the subject couch 104. If mounted relative to the couch, the movement of the couch may be used to derive the position of the camera 110 relative to the gantry, and hence relative to a scanning region of the scanner 120. The camera 110 may have a wide field of view so that it captures a full view of the subject couch 104, or at least the part of the patient couch over which regions of interest of the subject 102 will be positioned. The camera 110 for example comprises a fisheye lens with a wide field of view. The subject couch 104 (and the subject 102 on the subject couch 104) can be imaged either by a single camera as shown or by a small set of cameras.

In accordance with the invention, the processor 130 generates a cavity map describing distances between the visible surface and the body surface in a plurality of locations based on a comparison of a camera image and a scanned image taken at the point in time, in step 132. Motion of the visible surface of the subject 102 in at least one of the plurality of locations during the scanning session is detected in step 134. In step 136, a motion alert signal is generated, the motion alert signal indicating movement of the subject 102 based on the detected motion and the cavity map.

The motion alert signal may be provided as output for display on a display 140. This display 140 may be part of the medical scanner 120, or it may be a separate device such as a mobile device of a technician or it may be part of a remote operation center, to which the image data is transmitted by wired or wireless data transfer. Alternatively, the motion alert signal may be provided in the form of audio or haptic feedback to either the subject 102 or the operator of the medical scanner 120.

The processor 130 may be further configured to determine a motion confidence value indicative of a likelihood of movement of the body surface of the subject 102 based on the detected motion and the cavity map. Generating the motion alert signal may thus be based on the motion confidence value.

That is, generation of the motion alert signal may be dependent on the motion confidence value. The motion alert signal, in some cases, may be generated responsive to the motion confidence value satisfying a predetermined requirement. The predetermined requirement may be a percentage confidence in whether actual movement of the body of the subject has occurred.

Furthermore, the processor 130 may be configured to quantify an amount and/or direction of motion of the visible surface (i.e., the extent of motion of the visible surface). The motion alert signal may then be generated further based on the amount and/or direction of motion. That is, generation of the motion alert signal may occur dependent on the amount and/or movement direction, or may alternatively/additionally the amount and/or movement direction may be used to inform the generation of the motion alert signal.

In some cases, the detected motion of the visible surface of the subject 102 is located at a region of the subject 102 corresponding to a scan region of the medical scan. That is, the area of concern for movement (i.e., the area of the body that may impact the results of the scan if moved), will correspond to the scan region/the area inside the gantry. Thus, the motion may only be detected in this area for further analysis and potential generation of a motion alert signal.

In addition, the processor 130 may also be configured to generate a reliability map describing reliability of movement detection by the camera 110 at each of the plurality of locations based on the cavity map. Thus, the processor 130 is configured to generate the motion alert based on the detected motion and the reliability map. The reliability map essentially converts the distances between the body surface and the visible surface into a probability map, enabling a quick determination of an appropriate motion alert signal.

Furthermore, the reliability map can be used to identify areas and/or regions of the subject where motion may not be detected by the camera. Thus, an alert can be generated indicating such areas, so that a user or operator may be prompted to check such regions in scanner images for artifacts.

Also, the camera image and the scanner image can be registered, for example using a camera calibration with respect to the scanner. Of course, the scanner image may provide 3D coordinates of the body surface and of the materials (e.g., clothes/blankets) lying on the body. These, together with the calibration, allow projecting the segmented visible surface from the scanner image to the camera image. Therefore combining information from scanner image and camera image. This may ensure that an accurate mapping of the visible surface to the body surface.

To reiterate, the scanner image may be acquired before, during or after the camera images. In particular, the scanner image may be a low-dose 3D helical scan used as localizer scan (also known as 3D surview).

Finally, the processor 130 may also be configured to generate a visible surface map describing a shape of the visible surface of the subject 102 based on the camera image. That is, the camera image is segmented to obtain a surface map describing the shape of the visible surface. Similarly, the processor 130 may also generate a body surface map describing a shape of the body surface of the subject 102 based on the scanner image.

Accordingly, from these segmented images describing the shape, the cavity map may be generated by simply comparing the visible surface map to the body surface map (e.g., by overlaying the images and then performing image processing.

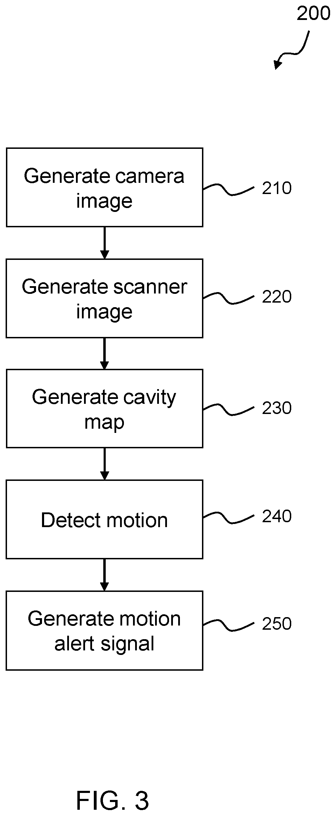

Fig. 3 presents a flow diagram of a method 200 for monitoring a subject 102 positioned on a subject couch 104 during a medical scan.

At step 210, a camera image of a visible surface at a point in time (e.g., before or during the medical scan) is generated. The visible surface corresponds to the subject 102 on the subject couch 104. That is, the visible surface is any surface that may expected to be impacted by movement of the subject during the medical scan (e.g., exposed skin, clothes, accessories, etc.).

At step 220, a scanner image of a body surface of the subject on the subject couch 104 is at the point in time is generated. In other words, the camera image and the scanner image are both images of the subject at a same point in time. Contrastingly, the camera image only has information on the position of the external surface of the subject, whereas the scanner image contains information on the position of the actual body of the subject (the part of the subject concerning the medical scan).

At step 230, a cavity map is generated. The cavity map describes distances between the visible surface and the body surface in a plurality of locations, and is generated based on a comparison of the camera image and the scanned image. Simply put, the cavity map contains a number of distances between the body and visible surface, and therefore indicates the presence or absence of cavities/gaps in each visible location.

At step 240, motion of the visible surface of the subject in at least one of the plurality of locations is detected during the scanning session. This may be achieved by the camera, or may instead be performed by a separate monitoring system.

Finally, at step 250, a motion alert signal is generated. The motion alert signal indicates movement of the subject, and is generated based on the detected motion and the cavity map. That is, generation of the motion alert signal is dependent on the detected motion in the context of the cavity map. For example, if there is detected movement in a location that has a cavity nearby as indicated by the cavity map, the motion alert signal may not be generated (or may be altered to indicate a lower chance of actual bodily movement).

Accordingly, the method is used to generate a motion alert signal that may more accurately indicate when motion has actual occurred. Indeed, the fact that current subject monitoring systems simply monitor movement of the external/visible surface (including clothes) of the subject means that false alarm may be prevalent. This method aims to overcome this issue by initially mapping any gaps between the visible/external/outer surface, and then referring to the mapping when movement is detected. As a result, the occurance of false alarms may be reduced, saving time and cognitive load for medical professionals.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A system (100) for monitoring a subject (102) positioned on a subject couch (104) during a medical scan, the system comprising:
a camera (110) for generating a camera image of a visible surface corresponding to the subject on the subject couch at a point in time;
a scanner (120) for generating a scanner image of a body surface of the subject on the subject couch at the point in time; and
a processor (130) for processing the camera image and the scanner image, and configured to:
generate (230) a cavity map describing distances between the visible surface and the body surface in a plurality of locations based on a comparison of the camera image and the scanned image;
detect (240) motion of the visible surface of the subject in at least one of the plurality of locations during the scanning session; and
generate (250) a motion alert signal indicating movement of the subject based on the detected motion and the cavity map.

2. The system of claim 1, wherein the camera (110) is further configured for generating camera images of the visible surface of the subject (102) during the medical scan, and
wherein the processor (130) is further configured to detect (240) motion of the visible surface of the subject based on the camera images of the visible surface of the subject during the scanning session.

3. The system of claim 1 or 2, wherein the scanner image further includes a covering surface corresponding to the subject on the subject couch.

4. The system of any of claims 1-3, wherein the processor (130) is further configured to determine a motion confidence value indicative of a likelihood of movement of the body surface of the subject (102) based on the detected motion and the cavity map, and wherein generating (250) the motion alert signal is based on the motion confidence value.

5. The system of claim 4, wherein the processor (130) is configured to generate (250) the motion alert signal responsive to the motion confidence value satisfying a predetermined requirement.

6. The system of any of claims 1-5, wherein the processor (130) is further configured to quantify an amount and/or direction of motion of the visible surface, and wherein the processor is configured to generate (250) the motion alert signal further based on the amount and/or direction of motion.

7. The system of any of claims 1-6, wherein the detected motion of the visible surface of the subject (102) is located at a region of the subject corresponding to a scan region of the medical scan.

8. The system of any of claims 1-7, wherein the processor (130) is further configured to generate a reliability map describing reliability of movement detection by the camera (110) at each of the plurality of locations based on the cavity map, and wherein the processor is configured to generate (250) the motion alert based on the detected motion and the reliability map.

9. The system of claim 8, wherein the processor is further configured to:
identify regions of the subject corresponding to values of the reliability map failing to meet a predetermined condition; and
generate an alert describing the identified regions.

10. The system of any of claims 1-9, wherein the processor (130) is further configured to:
generate a visible surface map describing a shape of the visible surface of the subject (102) based on the camera image;
generate a body surface map describing a shape of the body surface of the subject based on the scanner image; and
generate the cavity map (230) based on comparing the visible surface map to the body surface map.

11. The system of claim 10, wherein generating the visible surface map is further based on the scanner image.

12. The system of any of claims 1-11, wherein the processor is further configured to register the camera image and the scanner image based on a camera calibration with respect to the scanner and/or a scanner calibration with respect to the camera.

13. The system of any of claims 1-12, wherein the scanner image is an 3D surview scanner image, and wherein the point in time precedes initiation of the medical scan.

14. A method (200) for monitoring a subject (102) positioned on a subject couch (104) during a medical scan, the method comprising:
generating (210) a camera image of a visible surface corresponding to the subject on the subject couch at a point in time;
generating (220) a scanner image of a body surface of the subject on the subject couch at the point in time;
generating (230) a cavity map describing distances between the visible surface and the body surface in a plurality of locations based on a comparison of the camera image and the scanned image;
detecting (240) motion of the visible surface of the subject in at least one of the plurality of locations during the scanning session; and
generating (250) a motion alert signal indicating movement of the subject based on the detected motion and the cavity map.

15. A computer program comprising computer program code which is adapted, when said program is run on a computer, to implement the method (200) of any one of claims 10 to 14.
